# EUROPÄISCHE PATENTSCHRIFT

(11) **EP 0 920 239 B1**
(45) Veröffentlichungstag und Bekanntmachung des Hinweises auf die Patenterteilung: **03.12.2008**
(21) Anmeldenummer: 98100918.6
(22) Anmeldetag: 20.01.1998
(51) Int. Cl.: H04R 25/00

(54) **Fixationselement für ein implantierbares Mikrofon**
Anchoring means for implantable microphone
Dispositif d'ancrage pour microphone implantable

(30) Priorität: 26.11.1997 DE 19752447
(43) Veröffentlichungstag der Anmeldung: 02.06.1999
(73) Patentinhaber: PHONAK AG, 8712 Stäfa (CH)
(72) Erfinder: Leysieffer, Hans, Dr. Dipl.-Ing., 82024 Taufkirchen (DE); Baumann, Joachim W., Dipl.-Ing., 81679 München (DE); Lehner, Rolf Martin, Dipl.-Ing., 73734 Esslingen (DE); Müller, Gerd, Dr.rer.nat. Dipl.-Physiker, 85716 Unterschleissheim (DE); Reischl, Gabriele, Dipl.-Ing. (FH), 81541 München (DE)
(74) Vertreter: Schwan, Gerhard

(56) Entgegenhaltungen:
- WO-A-97/36457
- DE-A1- 3 707 161
- US-A- 5 277 694

## Beschreibung

Die Erfindung betrifft ein Fixationselement für ein implantierbares Mikrofon, das mit einem mit einer Schalleintrittsmembran versehenen zylinderförmigen Gehäuseteil in eine die hintere knöcherne Gehörgangswand durchquerende Bohrung einsetzbar ist und das beispielsweise einen Bestandteil einer teil- oder vollimplantierbaren Hörhilfe bilden kann.

Ein Ausführungsbeispiel eines Mikrofons der vorstehend genannten Art ist Gegenstand der früheren EP-Patentanmeldung 97 114 603 und in dem Aufsatz "Ein implantierbares Mikrofon für elektronische Hörimplantate" von H. Leysieffer et al., HNO 45: 816-827 (Oktober 1997) näher erläutert. Aus dem genannten Aufsatz ist es bekannt, das Mikrofongehäuse im Mastoid mittels Knochenzement mechanisch zu fixieren. Eine Zementfixierung ist jedoch mit zahlreichen Problemen behaftet. So kann der Knochenzement unter Umständen am Implantationsort unerwünschte Nebeneffekte auslösen. Die Handhabung ist angesichts der beengten Platzverhältnisse und der ungünstigen Sichtbedingungen am Implantationsort schwierig. Knochenzement kann unerwünscht auch an Stellen gelangen, wo er stört. Eine nachträgliche Korrektur der Mikrofonposition in der Bohrung der Gehörgangswand ist praktisch ausgeschlossen. Es wäre denkbar, das Mikrofongehäuse in die Bohrung der Gehörgangswand einzuschrauben. Klinische Experimente zeigen jedoch, daß dies wegen der geringen Dicke der knöchernen Gehörgangswand problematisch ist. Außerdem werden Schrauben im Bereich dieser Implantationsstelle häufig auch noch lange Zeit nach dem chirurgischen Eingriff schmerzhaft wahrgenommen.

Der Erfindung liegt die Aufgabe zugrunde, ein Fixationselement der eingangs genannten Art zu schaffen, das eine wesentlich vereinfachte und gleichwohl hoch präzise Implantation des Mikrofons unter Vermeidung der mit dem Einsatz von Knochenzement verbundenen Nachteile gestattet.

Diese Aufgabe wird mit einem Fixationselement für ein implantierbares Mikrofon, das mit einem mit einer Schalleintrittsmembran versehenen zylinderförmigen Gehäuseteil in eine die hintere knöcherne Gehörgangswand durchquerende Bohrung einsetzbar ist, gelöst, das erfindungsgemäß durch eine Manschette gekennzeichnet ist, die mit einem zylindrischen Manschettenteil den zylinderförmigen Gehäuseteil umschließt und die gegen die der Gehörgangshaut zugewendete Seite der Gehörgangswand anlegbare, vorspringende, elastische Flanschteile aufweist.

Das Fixationselement nach der Erfindung erlaubt es, das Mikrofongehäuse in die die knöcherne Gehörgangswand durchquerende Bohrung einfach einzuclipsen. Der Einsatz von Knochenzement wird vermieden. Die sich an die knöcherne Gehörgangswand anlegenden Flanschteile sorgen für eine exakte Ausrichtung des Mikrofongehäuses mit Bezug auf die Gehörgangswand. Zusammen mit dem mit der Bohrungswand in Eingriff stehenden zylindrischen Manschettenteil stellen sie eine sichere und langzeitstabile Halterung des implantierten Mikrofons sicher.

In weiterer Ausgestaltung der Erfindung ist die Manschette mit weiteren vorspringenden Flanschteilen versehen, die gegen die von der Gehörgangshaut abgewendete Seite der knöchernen Gehörgangswand anlegbar sind. Dadurch wird der Halt des Mikrofons im implantierten Zustand weiter verbessert. Ferner ist ein unbeabsichtigtes Kippen des Mikrofongehäuses gegenüber der Gehörgangswand während des Implantationsvorganges oder später zuverlässig ausgeschlossen.

Vorzugsweise ist die gesamte Manschette aus biokompatiblem elastischem Werkstoff gefertigt, der zweckmäßig aus der aus Silikonen und Polyurethanen bestehenden Gruppe ausgewählt ist. Ein solcher Werkstoff erlaubt eine begrenzte Verformung der Manschette beim Einsetzen in die Bohrung der knöchernen Gehörgangswand. Er ermöglicht es den Flanschteilen, sich gegen die betreffende Seite der Gehörgangswand einwandfrei anzulegen. Im Bereich des zylindrischen Manschettenteils kann auf einfache Weise für eine Klemmkraft gesorgt werden.

In weiterer Ausgestaltung der Erfindung kann die Manschette Teil einer Ummantelung sein, welche das Mikrofon mit Ausnahme der Schalleintrittsmembran mindestens zum größeren Teil und vorzugsweise ganz umschließt. Dadurch wird eine besonders sichere Verankerung der Manschette mit Bezug auf das Mikrofongehäuse erreicht.

Die Flanschteile, die sich im implantierten Zustand gegen die der Gehörgangshaut zugewendete Seite der Gehörgangswand anlegen sollen, lassen sich beim Implantieren mittels eines Werkzeugs in einer Lage halten, in welcher sie durch die Bohrung der Gehörgangswand hindurchgesteckt werden können. Das Implantieren des Mikrofons gestaltet sich aber besonders einfach, wenn in Weiterbildung der Erfindung eine Halterung vorgesehen ist, welche die gegen die der Gehörgangshaut zugewendete Seite der Gehörgangswand anlegbaren Flanschteile vor der Implantation entgegen einer elastischen Rückstellkraft der Flanschteile in einer das Durchstecken durch die Bohrung der Gehörgangswand erlaubenden umgebogenen Stellung hält. Dabei sind die gegen die der Gehörgangshaut zugewendete Seite der Gehörgangswand anlegbaren Flanschteile vorzugsweise so beschaffen und bemessen, daß sie nach Entfernen der Halterung in eine Stellung aufspringen, in welcher sie von dem zylindrischen Manschettenteil im wesentlichen radial abstehen. Der Operateur braucht bei einer solchen Ausbildung des Fixationselements nur den ummantelten zylinderförmigen Gehäuseteil des Mikrofons samt Halterung in die Bohrung der hinteren knöchernen Gehörgangswand einzusetzen und dann die Halterung unwirksam zu machen oder zu entfernen, um für den exakten und sicheren Sitz des Mikrofons am Implantationsort zu sorgen. Als Halterung kann beispielsweise einfach ein im Zuge der Implantation durchtrennbarer Faden vorgesehen sein, der zunächst die zugehörigen Flanschteile in einer das Implantieren nicht störenden Stellung hält und der anschließend die betreffenden Flanschteile aufgrund ihrer elastischen Eigenschaften in eine entspannte Lage übergehen läßt, in welcher sie ihre Haltefunktion übernehmen. Als Halterung kann gemäß einer vorteilhaften abgewandelten Ausführungsform auch eine auf die umgebogenen Flanschteile aufgesteckte Kappe vorgesehen sein.

Entsprechend einer weiteren Ausgestaltung der Erfindung sind die gegen die der Gehörgangshaut zugewendete Seite der Gehörgangswand anlegbaren Flanschteile ausgehend von dem zylindrischen Manschettenteil in Richtung auf diesen Manschettenteil geneigt. Dies trägt zu einem einwandfreien Anlegen der Flanschteile an die der Gehörgangshaut zugewendete Seite der Gehörgangswand bei, auch wenn die betreffende Oberfläche der Gehörgangswand aufgrund der anatomischen Gegebenheiten gewisse Unebenheiten aufweist. Aus dem gleichen Grund nimmt vorzugsweise die Wandstärke der gegen die der Gehörgangshaut zugewendete Seite der Gehörgangswand anlegbaren Flanschteile radial nach außen ab.

Die gegen die der Gehörgangshaut zugewendete Seite der Gehörgangswand anlegbaren Flanschteile können insbesondere als ein im entspannten Zustand ausgehend von dem zylindrischen Manschettenteil im wesentlichen radial abstehender Ringflansch ausgebildet sein. Grundsätzlich kommen jedoch auch andere geometrische Konfigurationen in Betracht, sofern nur einerseits die erforderliche Haltefunktion gewährleistet ist und andererseits die Flanschteile im Raum zwischen Gehörgangshaut und Gehörgangswand problemlos Platz finden.

Vorzugsweise sind die gegen die der Gehörgangshaut zugewendete Seite der Gehörgangswand anlegbaren Flanschteile mit Durchbrechungen versehen, die insbesondere die Aufgabe haben, einen Nährstoffübergang zwischen der knöchernen Gehörgangswand und der Gehörgangshaut im Bereich des Ringflanschs zu ermöglichen.

Es versteht sich, daß das erfindungsgemäße Fixationselement grundsätzlich für Mikrofone beliebiger Form, zum Beispiel auch für Mikrofone mit durchgehend zylindrischem Gehäuse, geeignet ist. Wenn das Mikrofon aber in an sich bekannter Weise (Aufsatz "Ein implantierbares Mikrofon für elektronische Hörimplantate" von H. Leysieffer et al., HNO 45: 816-827 (Oktober 1997)) mit einem mehrschenkligen Mikrofongehäuse versehen ist, bei dem ein erster Gehäuseschenkel den zylinderförmigen Gehäuseteil bildet und ein zweiter Gehäuseschenkel um eine etwa der Dicke der hinteren knöchernen Gehörgangswand entsprechende Strecke gegenüber der Ebene der Schalleintrittsmembran zurückversetzt ist, bildet vorteilhaft der zweite Gehäuseschenkel selbst oder ein den zweiten Gehäuseschenkel ummantelnder Teil des Fixationselements mindestens einen Teil der Flanschteile, die gegen die von der Gehörgangshaut abgewendete Seite der Gehörgangswand anlegbar sind. Dabei gehört zu den Flanschteilen, die gegen die von der Gehörgangshaut abgewendete Seite der Gehörgangswand anlegbar sind, vorzugsweise eine Schulter, die auf der dem zweiten Gehäuseschenkel diametral gegenüberliegenden Seite von dem zylindrischen Manschettenteil vorspringt.

Zweckmäßig ist mindestens die Manschette aus einem Werkstoff mit einer Shore A-Härte von 20 bis 70 gefertigt.

Bevorzugte Ausführungsbeispiele der Erfindung sind nachstehend unter Bezugnahme auf die beiliegenden Zeichnungen näher erläutert. Dabei zeigen:
- FIG. 1: eine schematische Darstellung eines in die hintere knöcherne Gehörgangswand implantierten Mikrofons, das mittels eines erfindungsgemäßen Fixationselements gehalten ist;
- FIG. 2: in größerem Maßstab eine Ansicht des bei dem Mikrofon nach FIG. 1 vorgesehenen Fixationselements in Blickrichtung von Pfeil A in FIG. 1;
- FIG. 3: eine Draufsicht auf das Fixationselement gemäß FIG. 2;
- FIG. 4: eine Schnittansicht des Fixationselements gemäß der Linie IV-IV in FIG. 3;
- FIG. 5: eine Detailansicht in noch größerem Maßstab des in FIG. 4 mit einem Kreis V angedeuteten Bereichs des Fixationselements;
- FIG. 6: eine Ansicht ähnlich FIG. 2, wobei jedoch der Ringflansch schematisch in dem Zustand dargestellt ist, in den er in Vorbereitung auf die Implantation gebracht wird;
- FIG. 7: eine Draufsicht ähnlich FIG. 3 für ein Fixationselement gemäß einer abgewandelten Ausführungsform;
- FIG. 8: eine Seitenansicht eines Fixationselements gemäß einer weiter abgewandelten Ausführungsform und
- FIG. 9: eine Ansicht ähnlich FIG. 6, wobei jedoch eine Kappe zum Zurückhalten der gegen die der Gehörgangshaut zugewendete Seite der Gehörgangswand anlegbaren Flanschteile vor und während der Implantation aufgesetzt ist.

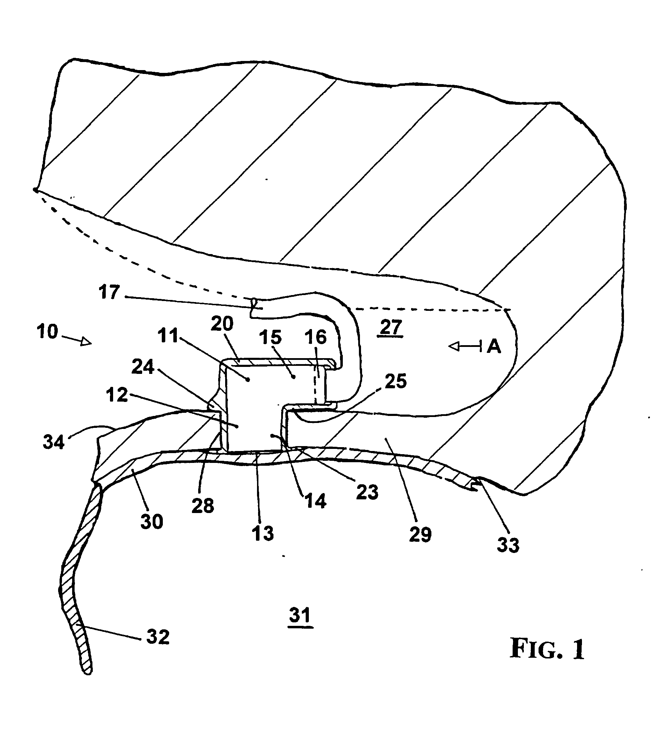

FIG. 1 zeigt schematisch ein Mikrofon 10, das Bestandteil einer teil- oder vollimplantierbaren Hörhilfe sein kann. Die Hörhilfe als solche kann in an sich bekannter Weise ausgebildet sein. Beispiele solcher Hörhilfen sind in dem Aufsatz "Aktive elektronische Hörimplantate für Mittel- und Innenohrschwerhörige - eine neue Ära der Ohrchirurgie" von H. P. Zenner et al., HNO 45: 749-757 (Oktober 1997) näher erläutert. Das Mikrofon 10 weist ein zweischenkliges Mikrofongehäuse 11 auf, welches in FIG. 1 zur Vereinfachung der Darstellung nur schematisch angedeutet und daher nicht im Schnitt veranschaulicht ist. In einem ersten Schenkel 12 des Mikrofongehäuses 11 ist eine Mikrofonkapsel untergebracht, und der Schenkel 12 bildet einen mit einer Schalleintrittsmembran 13 versehenen zylinderförmigen Gehäuseteil 14. Ein zweiter Gehäuseschenkel 15 nimmt eine schematisch bei 16 angedeutete elektrische Durchführung für eine elektrische Zuleitung 17 auf.

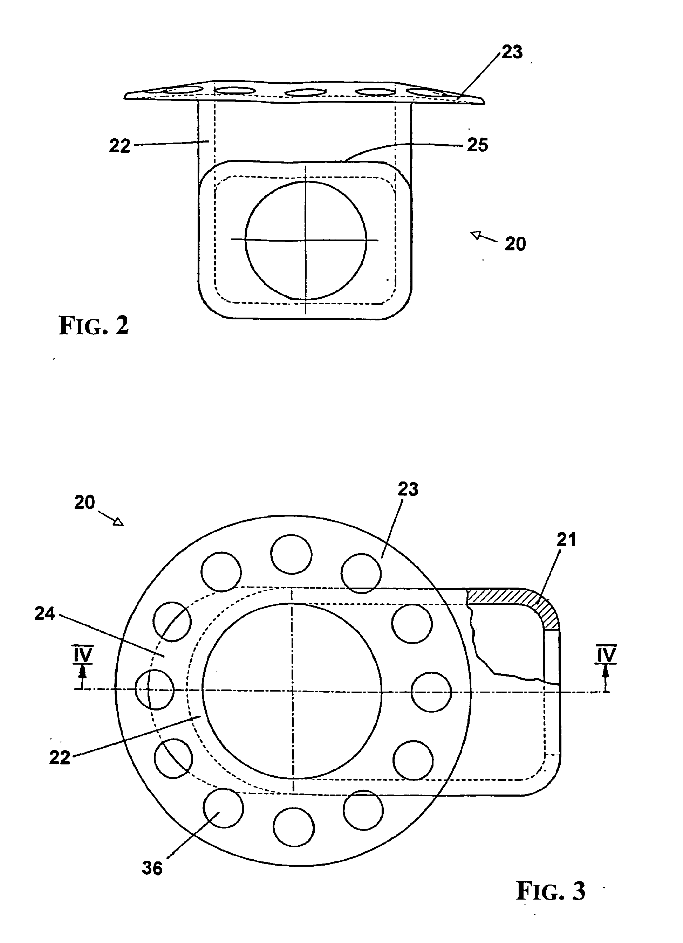

Das Mikrofon 10 ist mit einem Fixationselement 20 ausgestattet, das im veranschaulichten Ausführungsbeispiel eine das ganze Mikrofon aufnehmende Ummantelung bildet. Teil des Fixationselements 20 ist, wie im einzelnen in den Figuren 2 bis 5 dargestellt ist, eine Manschette 21, die mit einem zylindrischen Manschettenteil 22 den zylinderförmigen Gehäuseteil 14 umschließt. Zu der Manschette 21 gehören ferner Flanschteile 23, 24 und 25, die an beiden axialen Enden des zylindrischen Manschettenteils 22 radial nach außen vorspringen. Entsprechend FIG. 1 ist der zylinderförmige Gehäuseteil 14 zusammen mit dem zylindrischen Manschettenteil 22 von einer Mastoidhöhle 27 aus in eine Bohrung 28 einsetzbar, die eine hintere knöcherne Gehörgangswand 29 durchquert. In FIG. 1 sind der mit Gehörgangshaut 30 ausgekleidete Gehörgang bei 31 und das Trommelfell bei 32 angedeutet. Die Dimensionierung ist so gewählt, daß sich im implantierten Zustand des Mikrofons 10 die Flanschteile 23, 24 und 25 einerseits gegen die der Gehörgangshaut 30 zugewendete Seite 33 sowie andererseits gegen die von der Gehörgangshaut abgewendete Seite 34 der Gehörgangswand 29 anlegen und dabei die Schalleintrittsmembran 13 in direkten mechanischen Kontakt mit der Gehörgangshaut 30 kommt. Entsprechend ist der axiale Abstand ***a*** zwischen dem Flanschteil 23 und den Flanschteilen 24 und 25 so bemessen, daß er mit der mittleren Wandstärke der knöchernen Gehörgangswand 29 übereinstimmt. Der Abstand ***a*** liegt im allgemeinen im Bereich von 1,0 bis 2,5 mm. Vorzugsweise beträgt er etwa 1,6 mm. Die Schalleintrittsmembran 13 hat im Interesse einer guten Anlage an der Gehörgangshaut 30 vorzugsweise einen Durchmesser von weniger als 5,0 mm. Beispielsweise haben dementsprechend der zylinderförmige Gehäuseteil 14 einen Außendurchmesser von etwa 4,5 mm und der zylindrische Manschettenteil 22 einen Außendurchmesser von etwa 5,3 mm.

Bei der in den Figuren 1 bis 6 gezeigten Ausführungsform des Fixationselements 20 ist das mit der Schalleintrittsmembran 13 im wesentlichen bündig liegende Flanschteil 23 als Ringflansch ausgebildet, der im entspannten Zustand von dem gehörgangseitigen Ende des zylindrischen Manschettenteils 22 im wesentlich radial absteht. Der Ringflansch 23 ist mit einer Folge von in Umfangsrichtung verteilt angeordneten Durchbrechungen 36 versehen. Aufgabe der Durchbrechungen 36 ist es vor allem, einen Nährstofftransport zwischen der knöchernen Gehörgangswand 29 und der Gehörgangshaut 30 im Bereich des Ringflanschs 23 zuzulassen. Form und Anzahl der Durchbrechungen 36 sind unwesentlich, solange die Durchbrechungen diesen Zweck erfüllen und die notwendige mechanische Festigkeit des Ringflanschs 23 erhalten bleibt.

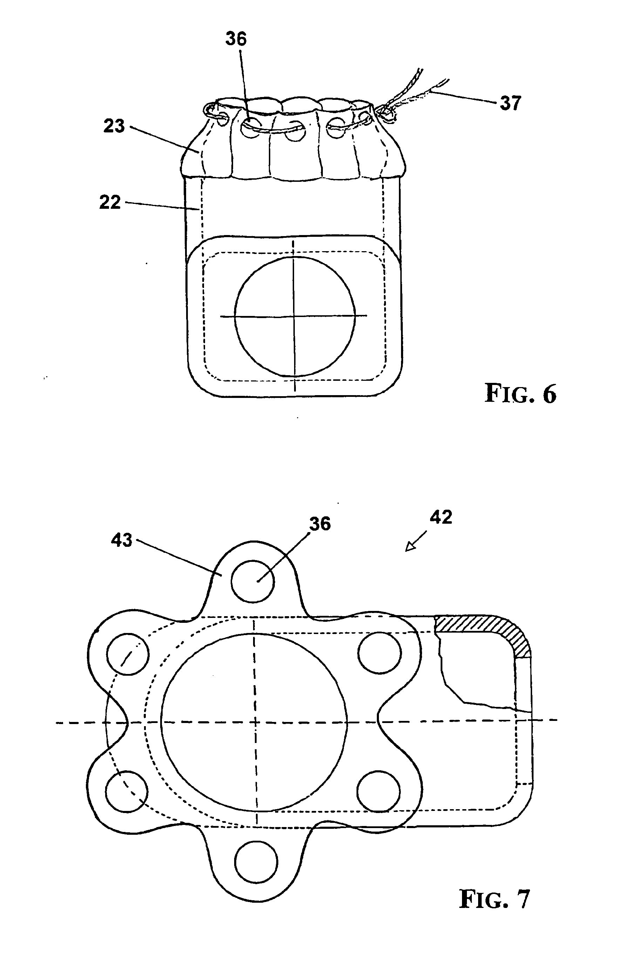

Vor der Implantation ist durch die Folge der Durchbrechungen 36 ein chirurgischer Faden 37 gefädelt und beispielsweise durch Verdrillen, Verknoten oder dergleichen in einem Zustand temporär fixiert, bei dem der Ringflansch 23 entsprechend FIG. 6 in axialer Richtung weg von den Flanschteilen 24 und 25 umgelegt und zusammengezogen ist. In dieser Form läßt sich der von dem zylindrischen Manschettenteil 22 umgebene zylinderförmige Gehäuseteil 14 durch die Bohrung 28 in der Gehörgangswand 30 hindurchstecken. Wird dann der Faden 37 durchtrennt, kehrt der Ringflansch 23 in seine entspannte Form gemäß den Figuren 1 bis 4 zurück. Um ein solches Verhalten zu erzwingen, ist mindestens der den Ringflansch 23 bildende Teil des Fixationselements 20 elastisch ausgebildet.

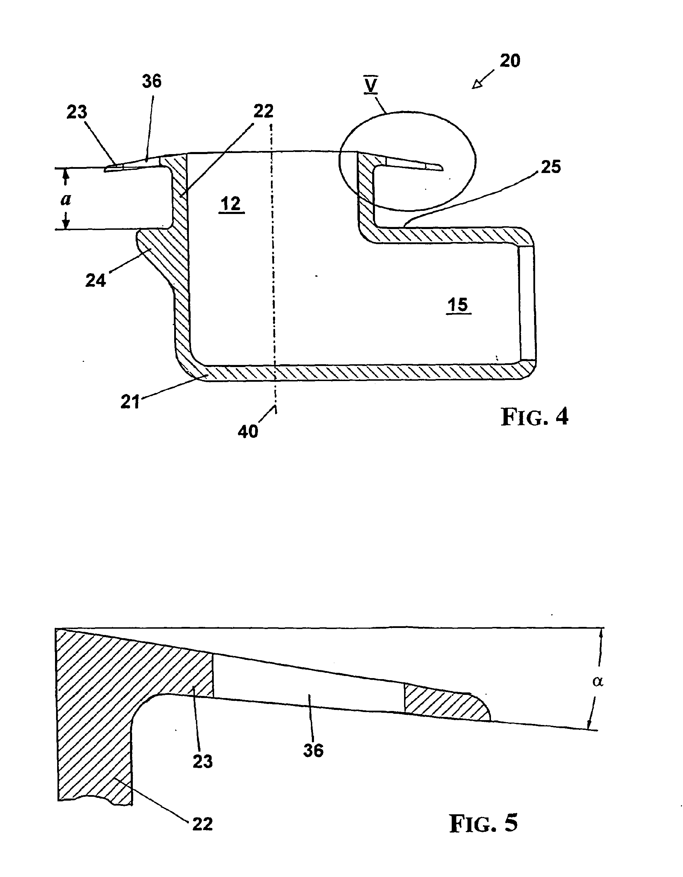

Der Ringflansch 23 soll sich im implantierten Zustand (FIG. 1) an die der Gehörgangshaut 30 zugewendete Seite 33 der Gehörgangswand 30 ungeachtet von Unebenheiten dieser Wandfläche möglichst eng anschmiegen. Um dies zu fördern, sind, neben einer geeigneten Stoffauswahl für mindestens den Ringflansch 23, bei der veranschaulichten Ausführungsform drei Maßnahmen vorgesehen, die besonders deutlich aus FIG. 5 zu erkennen sind. Zum einen ist die Wandstärke des Ringflanschs 23 so weit reduziert, wie dies ohne Beeinträchtigung der Haltefunktion des Ringflanschs möglich ist. Des weiteren ist der Ringflansch 23 ausgehend von dem zylindrischen Manschettenteil 22 in Richtung auf die Flanschteile 24, 25 geneigt. Schließlich nimmt die Wandstärke des Ringflanschs 23 radial nach außen ab. Die Wandstärke des Ringflanschs 23 kann dabei zweckmäßig von etwa 0,1 bis 0,2 mm nahe dem Manschettenteil 22 auf etwa 0,03 bis 0,1 mm am Außenrand des Ringflanschs 23 reduziert sein. Der Neigungswinkel α des Ringflanschs 23 kann im Bereich von 0 bis 20 Grad, vorzugsweise bei etwa 5 Grad, liegen.

Der Flanschteil 25 wird von dem Teil der Ummantelung (Fixationselement 20) gebildet, welcher die dem Ringflansch 23 zugewendete Seite des Gehäuseschenkels 15 überdeckt. Bei dem Flanschteil 24 handelt es sich um eine Schulter, die auf der dem Gehäuseschenkel 15 diametral gegenüberliegenden Seite von dem zylindrischen Manschettenteil 22 vorspringt (siehe insbesondere Figuren 3 und 4).

Bei der Implantation des mit dem Fixationselement 20 ummantelten Mikrofons 10 sorgen die Flanschteile 23, 24 und 25 für eine Ausrichtung der Schalleintrittsmembran 13 und des Gehäuseschenkels 15 parallel zu der knöchernen Gehörgangswand 29 in einer Position, in welcher die Schalleintrittsmembran 13 im wesentlichen bündig zu der der Gehörgangshaut 30 zugewendeten Seite 33 der Gehörgangswand 29 liegt. Auch einem Verkippen des Mikrofons 10, zum Beispiel durch eine beim Implantationsvorgang versehentlich auf das Mikrofongehäuse 11 und/oder die Zuleitung 17 ausgeübte Kraft, ist wirkungsvoll vorgebeugt. Das Fixationselement 20 hält das Mikrofon sicher am Implantationsort, ohne daß zusätzliche Maßnahmen oder Mittel, zum Beispiel Knochenzement, erforderlich werden. Im Bedarfsfall läßt sich das Mikrofon während der Implantation, nach Einbringen des zylindrischen Manschettenteils 22 in die Bohrung 28, um die in FIG. 4 bei 40 angedeutete Achse des zylinderförmigen Gehäuseteils 14 drehen.

Das Fixationselement 20 ist zweckmäßig ein das Mikrofongehäuse 11 aufnehmendes Spritzteil. Als Werkstoff für das Fixationselement 20 eignet sich insbesondere ein biokompatibler Kunststoff mit einer Shore A-Härte von 20 bis 70. Dabei kann es sich vor allem um Silikone oder Polyurethane handeln.

Die abgewandelte Ausführungsform des Fixationselements 42 gemäß FIG. 7 unterscheidet sich von dem zuvor erläuterten Fixationselement 20 im wesentlichen nur dadurch, daß die im implantierten Zustand zwischen der Gehörgangswand 29 und der Gehörgangshaut 30 liegenden Flanschteile nicht von einem Ringflansch 23, sondern von einer Folge von in Umfangsrichtung des zylindrischen Manschettenteils verteilten Flanschsegmenten 43 gebildet werden. Dabei können die Flanschsegmente 43, wie dargestellt, mit Durchbrechungen 36 versehen sein. Solche Durchbrechungen können aber auch entbehrlich sein, insbesondere dann, wenn schon aufgrund der Form der Flanschteile ein Nährstofftransport zwischen Gehörgangswand 29 und Gehörgangshaut 30 gewährleistet ist.

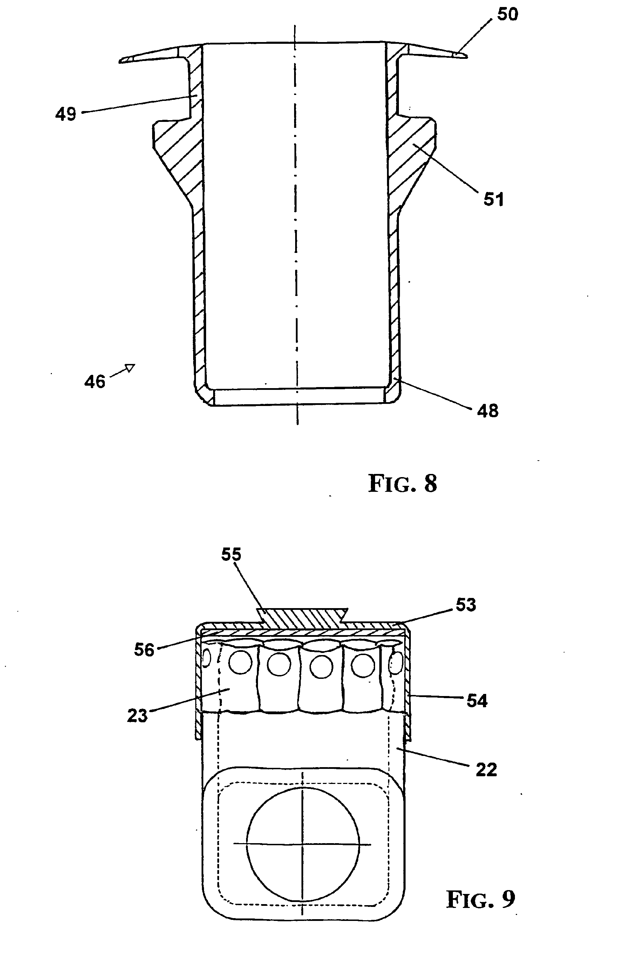

FIG. 8 zeigt eine weiter abgewandelte, insgesamt mit 46 bezeichnete Ausführungsform des Fixationselements, die sich für Mikrofone mit einschenkligem, zylinderförmigem Mikrofongehäuse eignet. Das Fixationselement 46 weist wiederum ein Manschette 48 auf, die mit einem zylindrischen Manschettenteil 49 einen zylinderförmigen Teil des Mikrofongehäuses umschließt und die gegen die in FIG. 1 dargestellte, der Gehörgangshaut 30 zugewendete Seite 33 sowie die von der Gehörgangshaut abgewendete Seite 34 der Gehörgangswand 29 anlegbare vorspringende Flanschteile 50 beziehungsweise 51 aufweist. Der Flanschteil 50 kann insbesondere entsprechend dem Flanschring 23 der Figuren 2 bis 6 ausgelegt oder von Flanschsegmenten 43 gemäß FIG. 7 gebildet sein. Der Flanschteil 51 kann zweckmäßig als ringförmige Schulter ausgebildet sein.

FIG. 9 zeigt eine Ansicht ähnlich FIG. 6, wobei jedoch anstelle des Fadens 37 eine Kappe 53 zum Zurückhalten der gegen die Seite 33 der Gehörgangswand 29 anlegbaren Flanschteile 23, 43 beziehungsweise 50 vor und während der Implantation vorgesehen ist. Die Kappe 53 ist mindestens im Bereich ihres hohlzylindrischen Teils 54 so dünnwandig ausgeführt, daß sie zusammen mit dem zylindrischen Manschettenteil 22 beziehungsweise 49 in die Bohrung 28 gesteckt und dann unter Freigabe der Flanschteile 23, 43 beziehungsweise 50 vom Gehörgang 31 aus abgezogen werden kann. Um dabei das Fassen der Kappe 53 mittels einer Pinzette oder dergleichen zu erleichtern, trägt die Kappe an ihrer geschlossenen Seite einen Nippel 55. In den hohlzylindrischen Teil 54 der Kappe 53 ist zweckmäßig eine relativ steife und harte Platte 56 eingesetzt. Der Kappenteil 54 ist vorzugsweise in begrenztem Umfang elastisch dehnbar. Der Kappenwerkstoff muß sterilisierbar sein; ansonsten kommt eine Vielzahl von Werkstoffen, insbesondere Metallen und Kunststoffen, in Betracht. Die Kappe 53 sorgt, vor allem wenn sie mit der Platte 56 ausgestattet ist, vor der Implantation für einen hervorragenden Schutz der Schalleintrittsmembran 13.

## Patentansprüche

1. Fixationselement für ein implantierbares Mikrofon (10), das mit einem mit einer Schalleintrittsmembran (13) versehenen zylinderförmigen Gehäuseteil (14) in eine die hintere knöcherne Gehörgangswand (29) durchquerende Bohrung (28) einsetzbar ist, **gekennzeichnet durch** eine Manschette (21, 48), die mit einem zylindrischen Manschettenteil (22, 49) den zylinderförmigen Gehäuseteil umschließt und die gegen die der Gehörgangshaut (30) zugewendete Seite (33) der Gehörgangswand anlegbare, vorspringende, elastische Flanschteile (23, 43, 50) aufweist.

2. Fixationselement nach Anspruch 1, **dadurch gekennzeichnet, daß** die Manschette (21, 48) mit weiteren vorspringenden Flanschteilen (24, 25, 51) versehen ist, die gegen die von der Gehörgangshaut (30) abgewendete Seite (33) der knöchernen Gehörgangswand (29) anlegbar sind.

3. Fixationselement nach Anspruch 1 oder 2, **dadurch gekennzeichnet, daß** die gesamte Manschette (21, 48) aus biokompatiblem elastischem Werkstoff gefertigt ist.

4. Fixationselement nach einem der vorhergehenden Ansprüche, **dadurch gekennzeichnet, daß** die Manschette (21, 48) Teil einer Ummantelung ist, welche das Mikrofon (10) mit Ausnahme der Schalleintrittsmembran (13) mindestens zum größeren Teil, und vorzugsweise ganz, umschließt.

5. Fixationselement nach einem der vorhergehenden Ansprüche, **gekennzeichnet durch** eine Halterung (37, 53), welche die gegen die der Gehörgangshaut (30) zugewendete Seite (33) der Gehörgangswand (29) anlegbaren Flanschteile (23, 43, 50) vor der Implantation entgegen einer elastischen Rückstellkraft der Flanschteile in einer das Durchstecken **durch** die Bohrung (28) der Gehörgangswand erlaubenden umgebogenen Stellung hält.

6. Fixationselement nach Anspruch 5, **dadurch gekennzeichnet, daß** die gegen die der Gehörgangshaut (30) zugewendete Seite (33) der Gehörgangswand (29) anlegbaren Flanschteile (23, 43, 50) so beschaffen und bemessen sind, daß sie nach Entfernen der Halterung (37, 53)) in eine Stellung aufspringen, in welcher sie von dem zylindrischen Manschettenteil (22, 49) im wesentlichen radial abstehen.

7. Fixationselement nach den Ansprüchen 5 und 6, **dadurch gekennzeichnet, daß** als Halterung ein im Zuge der Implantation durchtrennbarer Faden (37) vorgesehen ist.

8. Fixationselement nach den Ansprüchen 5 und 6, **dadurch gekennzeichnet, daß** als Halterung eine auf die umgebogenen Flanschteile (23, 43, 50) aufgesteckte Kappe (53) vorgesehen ist.

9. Fixationselement nach einem der vorhergehenden Ansprüche, **dadurch gekennzeichnet, daß** die gegen die der Gehörgangshaut (30) zugewendete Seite (33) der Gehörgangswand (29) anlegbaren Flanschteile (23, 43, 50) ausgehend von dem zylindrischen Manschettenteil (22, 49) in Richtung auf diesen Manschettenteil geneigt sind.

10. Fixationselement nach einem der vorhergehenden Ansprüche, **dadurch gekennzeichnet, daß** die Wandstärke der gegen die der Gehörgangshaut (30) zugewendete Seite (33) der Gehörgangswand (29) anlegbaren Flanschteile (23, 43, 50) radial nach außen abnimmt.

11. Fixationselement nach einem der vorhergehenden Ansprüche, **dadurch gekennzeichnet, daß** die gegen die der Gehörgangshaut (30) zugewendete Seite (33) der Gehörgangswand (29) anlegbaren Flanschteile (23) als im entspannten Zustand ausgehend von dem zylindrischen Manschettenteil (22, 49) im wesentlichen radial abstehender Ringflansch ausgebildet sind.

12. Fixationselement nach einem der vorhergehenden Ansprüche, **dadurch gekennzeichnet, daß** die gegen die der Gehörgangshaut (30) zugewendete Seite (33) der Gehörgangswand (29) anlegbaren Flanschteile (23, 43, 50) mit Durchbrechungen (36) versehen ist.

13. Fixationselement nach einem der Ansprüche 2 bis 12 für ein Mikrofon (10) mit mehrschenkligem Mikrofongehäuse (11), wobei ein erster Gehäuseschenkel (12) den zylinderförmigen Gehäuseteil (14) bildet und ein zweiter Gehäuseschenkel (15) um eine etwa der Dicke der hinteren knöchernen Gehörgangswand entsprechende Strecke (a) gegenüber der Ebene der Schalleintrittsmembran (13) zurückversetzt ist, **dadurch gekennzeichnet, daß** der zweite Gehäuseschenkel (15) selbst oder ein den zweiten Gehäuseschenkel ummantelnder Teil des Fixationselements (20) mindestens einen Teil (25) der Flanschteile (24, 25) bildet, die gegen die von der Gehörgangshaut (30) abgewendete Seite (34) der Gehörgangswand (29) anlegbar sind.

14. Fixationselement nach Anspruch 13, **dadurch gekennzeichnet, daß** zu den Flanschteilen (24, 25), die gegen die von der Gehörgangshaut (30) abgewendete Seite (34) der Gehörgangswand (29) anlegbar sind, eine Schulter (24) gehört, die auf der dem zweiten Gehäuseschenkel (15) diametral gegenüberliegenden Seite von dem zylindrischen Manschettenteil (22) vorspringt.

15. Fixationselement nach einem der vorhergehenden Ansprüche, **dadurch gekennzeichnet, daß** mindestens die Manschette (21, 48) aus einem Werkstoff mit einer Shore A-Härte von 20 bis 70 gefertigt ist.

16. Fixationselement nach einem der vorhergehenden Ansprüche, **dadurch gekennzeichnet, daß** mindestens die Manschette (21, 48) aus einem Werkstoff gefertigt ist, der aus der aus Silikonen und Polyurethanen bestehenden Gruppe ausgewählt ist.

## Claims

1. Fixation element for an implantable microphone (10) having a cylindrical housing part (14) provided with a sound inlet membrane (13), which microphone is insertable into a hole (28) which crosses a rear bony wall (29) of the auditory canal, **characterized by** a sleeve (21, 48) which has a cylindrical sleeve part (22, 49) for surrounding the cylindrical housing part and which has projecting elastic flange parts (23, 43, 50) which are engageable against that side (33) of the wall of the auditory canal which faces the skin (30) of the auditory canal.

2. Fixation element according to claim 1, wherein the sleeve (21, 48) is provided with additional projecting flange parts (24, 25, 51) which are engageable against that side (33) of the bony wall (29) of the auditory canal which faces away from the skin (30) of the auditory canal.

3. Fixation element according to one of claims 1 and 2, wherein the entire sleeve (21, 48) is made of a biocompatible elastic material.

4. Fixation element according to one of the preceding claims, wherein the sleeve (21, 48) forms part of a casing which at least primarily, and preferably entirely, surrounds the microphone (10) except for the sound inlet membrane (13).

5. Fixation element according to one of the preceding claims, further comprising a holder (37, 53) which holds the flange parts (23, 43, 50), which are engageable against that side (33) of the wall (29) of the auditory canal which faces the skin (30) of the auditory canal, in a bent position against an elastic restoring force of the flange parts before implantation and which allows insertion of the flange parts through the hole (28) of the wall of the auditory canal.

6. Fixation element according to claim 5, wherein the flange parts (23, 43, 50), which are engageable against that side (33) of the wall (29) of the auditory canal facing the skin (30) of the auditory canal, are composed and dimensioned such that after removal of the holder (37, 53) they spring into a position in which they project essentially radially away from the cylindrical sleeve part (22, 49).

7. Fixation element according to one of claims 5 and 6, wherein the holder is a thread (37) which is severable in the course of implantation.

8. Fixation element according to one of claims 5 and 6, wherein the holder is a cap (53) which is placeable on the flange part (23, 43, 50) in the bent position thereof.

9. Fixation element according to one of the preceding claims, wherein the flange parts (23, 43, 50), which are engageable against that side (33) of the wall (29) of the auditory canal which faces the skin (30) of the auditory canal, are angled, starting from the cylindrical sleeve part (22, 49), toward that sleeve part.

10. Fixation element according to one of the preceding claims, wherein the wall thickness of the flange parts (23, 43, 50), which are engageable against that side (33) of the wall (29) of the auditory canal which faces the skin (30) of the auditory canal, decreases in a radially outward direction.

11. Fixation element to one of the preceding claims, wherein the flange parts (23), which are engageable against that side (33) of the wall (29) of the auditory canal which faces the skin (30) of the auditory canal, are formed as an annular flange which projects away from the cylindrical sleeve part (22, 49) in an essentially radial direction in an unstressed state.

12. Fixation element according to one of the preceding claims, wherein the flange parts (23, 43, 50), which are engageable against that side (33) of the wall (29) of the auditory canal which faces the skin (3) of the auditory canal, are provided with openings (36).

13. Fixation element according to one of claims 2 to 12, for a microphone (10) with a multi-leg microphone housing (11) having a first housing leg (12) forming the cylindrical microphone housing part (14) and a second housing leg (15) which is set back by a distance a corresponding to the thickness of the rear bony wall of the auditory canal relative to the plane of the sound inlet membrane (13), wherein the second housing leg (15) itself or a part of the fixation element (20) jacketing the second housing leg forms at least a part (25) of the flange parts (24, 25) which are engageable against that side (34) of the wall (29) of the auditory canal which faces away from the skin (30) of the auditory canal.

14. Fixation element according to claim 13, wherein the flange parts (24, 25), which are engageable against that side (34) of the wall (29) of the auditory canal which faces away from the skin (30) of the auditory canal, comprise a shoulder (24) which projects from the cylindrical sleeve part (22) on the side diametrically opposite from the second housing leg (15).

15. Fixation element according to one of the preceding claims, wherein at least the sleeve (21, 48) is made from a material having a shore A hardness from 20 to 70.

16. Fixation element according to one of the preceding claims, wherein at least the sleeve (21, 48) is made from a material selected from the group consisting of silicones and polyurethanes.

## Revendications

1. Elément de fixation pour un microphone (10) implantable, qui peut être inséré avec une partie de boîtier (14) cylindrique, doté d'une membrane d'entrée de son (13) dans un perçage (28) traversant la paroi arrière osseuse du conduit auditif (29), **caractérisé par** un manchon (21, 48), qui entoure avec une partie de manchon (22, 49) cylindrique la partie de boîtier cylindrique et qui présente des parties de bride (23, 43, 50) élastiques, saillantes, pouvant être appliquées contre le côté (33), tourné vers la peau du conduit auditif (30), de la paroi du conduit auditif.

2. Elément de fixation selon la revendication 1, **caractérisé en ce que** le manchon (21, 48) est doté d'autres parties de bride (24, 25, 51) saillantes, qui peuvent être appliquées contre le côté (33), opposé à la peau du conduit auditif (30), de la paroi osseuse du conduit auditif (29).

3. Elément de fixation selon la revendication 1 ou 2, **caractérisé en ce que** le manchon (21, 48) complet est fabriqué à base de matériau élastique biocompatible.

4. Elément de fixation selon l'une quelconque des revendications précédentes, **caractérisé en ce que** le manchon (21, 48) fait partie d'une enveloppe qui enveloppe, au moins pour la majeure partie, et de préférence complètement, le microphone (10) à l'exception de la membrane d'entrée de son (13).

5. Elément de fixation selon l'une quelconque des revendications précédentes, **caractérisé par** une fixation (37, 53), qui maintient les parties de bride (23, 43, 50) pouvant être appliquées contre le côté (33), tourné vers la peau du conduit auditif (30), de la paroi du conduit auditif (29) avant l'implantation dans le sens contraire à une force de rappel élastique des parties de bride dans une position repliée autorisant la traversée du perçage (28) de la paroi du conduit auditif.

6. Elément de fixation selon la revendication 5, **caractérisé en ce que** les parties de bride (23, 43, 50) pouvant être appliquées contre le côté (33), tourné vers la peau du conduit auditif (30), de la paroi du côté auditif (29) sont conçues et dimensionnées de telle sorte que, après l'enlèvement de la fixation (37, 53), elles s'ouvrent dans une position dans laquelle elles dépassent essentiellement radialement de la partie de manchon (22, 49) cylindrique.

7. Elément de fixation selon les revendications 5 et 6, **caractérisé en ce qu'**un fil (37) séparable est prévu comme fixation en cours d'implantation.

8. Elément de fixation selon les revendications 5 et 6, **caractérisé en ce qu'**un capot (53) emboîté sur les parties de bride (23, 43, 50) repliées est prévu comme fixation.

9. Elément de fixation selon l'une quelconque des revendications précédentes, **caractérisé en ce que** les parties de bride (23, 43, 50) pouvant être appliquées contre le côté (33), tourné vers la peau du conduit auditif (30), de la paroi du conduit auditif (29) sont inclinées à partir de la partie de manchon (22, 49) cylindrique en direction de cette partie de manchon.

10. Elément de fixation selon l'une quelconque des revendications précédentes, **caractérisé en ce que** l'épaisseur de paroi des parties de bride (23, 43, 50) pouvant être appliquées contre le côté (33), tourné vers la peau du conduit auditif (30), de la paroi du conduit auditif (29) diminue radialement vers l'extérieur.

11. Elément de fixation selon l'une quelconque des revendications précédentes, **caractérisé en ce que** les parties de bride (23) pouvant être appliquées contre le côté (33), tourné vers la peau du conduit auditif (30), de la paroi du conduit auditif (29) sont réalisées sous forme de bride annulaire dépassant sensiblement radialement dans l'état détendu à partir de la partie de manchon (22, 49) cylindrique.

12. Elément de fixation selon l'une quelconque des revendications précédentes, **caractérisé en ce que** les parties de bride (23, 43, 50) pouvant être appliquées contre le côté (33), tourné vers la peau du conduit auditif (30), de la paroi du conduit auditif (29) sont dotées de débouchages (36).

13. Elément de fixation selon l'une quelconque des revendications 2 à 12 pour un microphone (10) avec boîtier de microphone (11) multibranches, une première branche de boîtier (12) formant la partie de boîtier (14) cylindrique et une seconde branche de boîtier (15) étant reculée d'un tronçon (a) correspondant à peu près à l'épaisseur de la paroi arrière osseuse du conduit auditif par rapport au plan de la membrane d'entrée de son (13), **caractérisé en ce que** la seconde branche de boîtier (15) même ou une partie, enveloppant la seconde branche de boîtier, de l'élément de fixation (20) forme au moins une partie (25) des parties de bride (24, 25) qui peuvent être appliquées contre le côté (34), opposé à la peau du conduit auditif (30), de la paroi du conduit auditif (29).

14. Elément de fixation selon la revendication 13, **caractérisé en ce que** les parties de bride (24, 25), qui peuvent être appliquées contre le côté (34), opposé à la peau du conduit auditif (30), de la paroi du conduit auditif (29) comprennent un épaulement (24) qui fait saillie sur le côté, diamétralement opposé à la seconde branche de boîtier (15), de la partie de manchon (22) cylindrique.

15. Elément de fixation selon l'une quelconque des revendications précédentes, **caractérisé en ce que** au moins le manchon (21, 48) est fabriqué à base d'un matériau présentant une dureté Shore A de 20 à 70.

16. Elément de fixation selon l'une quelconque des revendications précédentes, **caractérisé en ce que** au moins le manchon (21, 48) est fabriqué à base d'un matériau qui est choisi dans le groupe constitué de silicones et de polyuréthannes.
